(19) Europäisches Patentamt
European Patent Office
Office européen des brevets

(11) EP 1 413 627 A1

(12) # EUROPEAN PATENT APPLICATION

(43) Date of publication:
**28.04.2004 Bulletin 2004/18**

(51) Int Cl.$^7$: **C12P 17/04**, C12P 41/00

(21) Application number: **03024203.6**

(22) Date of filing: **21.10.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **25.10.2002 IT MI20022273**

(71) Applicant: **PRIME EUROPEAN THERAPEUTICALS S.p.A.,**
**in abbreviated form EUTICALS S.p.A.**
**20075 SANGRATO (LODI) (IT)**

(72) Inventors:
- **Molinari, Francesco**
  **20075 Sangrato (Lodi) (IT)**
- **Gandolfi, Raffaella**
  **20075 Sangrato (Lodi) (IT)**

(74) Representative: **Minoja, Fabrizio, Dr.**
**Bianchetti Bracco Minoja S.r.l.**
**Via Rossini, 8**
**20122 Milano (IT)**

(54) **Enzymatic synthesis of (S)-1,2-O-isopropylidene glycerol**

(57) This invention relates to a process for the synthesis of (S)-1,2-O-isopropylidene glycerol by hydrolysis of a racemic mixture of (R,S)-1,2-O-isopropylidene glycerol acetate catalysed by *Kluyveromyces marxianus*, recovery of the unreacted (R)-1,2-O-isopropylidene glycerol acetate, and hydrolysis thereof.

EP 1 413 627 A1

## Description

### FIELD OF INVENTION

This invention relates to the synthesis of 1,2-O-isopropylidene glycerol, and in particular to the synthesis of (S)-1,2-O-isopropylidene glycerol by enzymatic hydrolysis of (R,S)-1,2-O-isopropylidene glycerol acetate.

### BACKGROUND OF THE INVENTION

1,2-O-isopropylidene glycerol or solketal (I), in both enantiomeric forms, is used as a precursor of glycerol in the synthesis of molecules of pharmaceutical interest, such as β-blockers, glycerophospholipids, PAF (Platelet Aggregating Factor), aryloxypropanolamines and other products used in the treatment of epilepsy and hypertension (J. Jurczac, S. Pikul, T. Bauer, *Tetrahedron*, 1986, 42, 447).

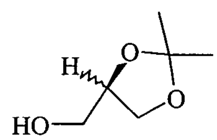

(I)

The optically active forms of (I) can be obtained by reduction of homochiral aldehydes.

(S)-isopropylidene glyceraldehyde can be obtained (scheme 1) from D-mannitol, doubly protected as acetonide, by means of degradation catalysed by $Pb(OAc)_4$ (Hanson R.M. Chem. Rev. 1991; 91:437).

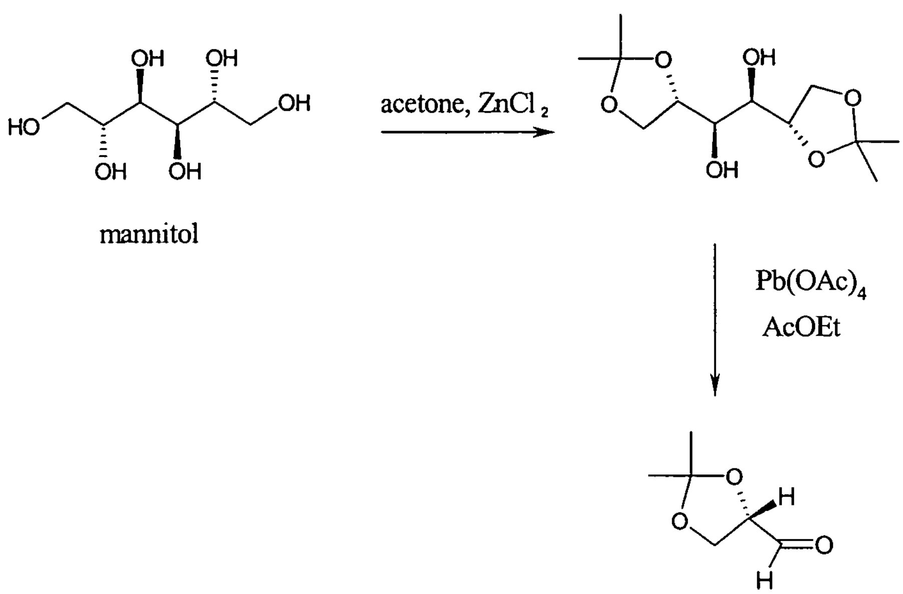


mannitol

(S)-isopropylidene glyceraldeyde

**Scheme 1**

(R) isopropylidene glyceraldehyde can be obtained (scheme 2) from ascorbic acid protected as acetonide (Hanson R.M. Chem. Rev. 1991; 91:437).

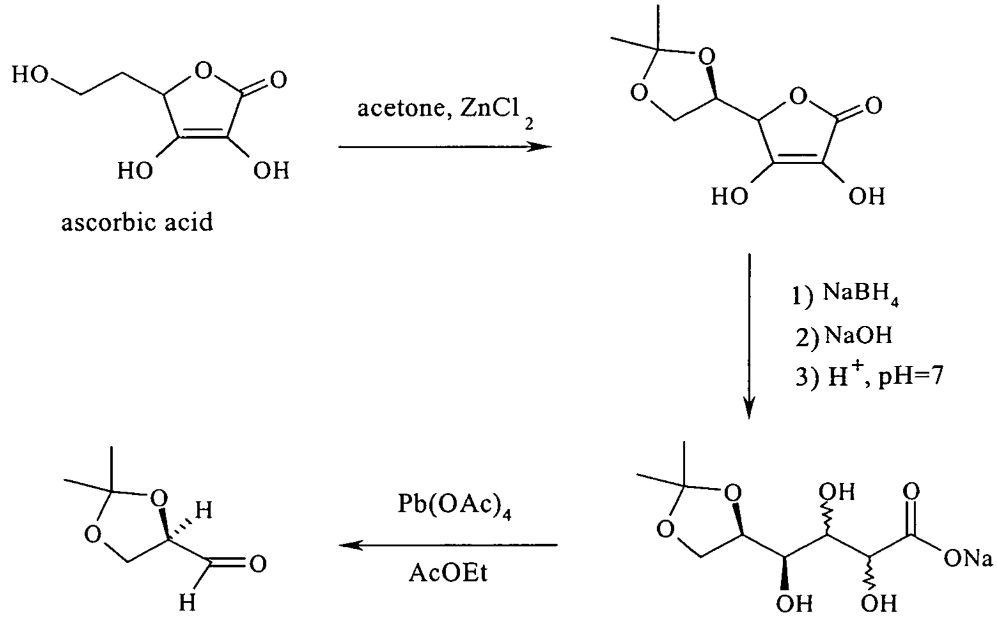


**Scheme 2**

Despite the simplicity of the reactions, the costs of production are rather high, so research has focused on developing biotechnology methods which allow the production of enantiomerically enriched chiral synthons with high yields and low costs. The use of biocatalysts (whole cells or isolated enzymes) in the synthesis of molecules of pharmaceutical interest offers a number of advantages over conventional synthesis processes (Faber K., Springer-Verlag, *Biotrasformations in Organic Chemistry*, 1992, pg. 3), especially:

- high catalytic efficiency (biocatalysts can increase the rate of a reaction by a factor equal to or greater than $10^{12}$ compared with the non-catalysed reaction, these values often being much higher than those obtainable with chemical catalysts);
- environmental compatibility (biocatalysts are non-pathogenic biological systems and therefore environment-friendly; moreover, they are completely degraded by the environment, unlike metal chemocatalysts, which can be difficult to dispose of);
- possibility of working under mild reaction conditions (biocatalysts usually operate in aqueous systems, in a pH range of 5 to 8, at a temperature of 20 to 40C° and at atmospheric pressure);
- high chemo-, regio- and stereoselectivity of the catalysed reactions.

The choice of biocatalyst (isolated enzyme or whole cell), the physical state thereof (immobilised or free) and the reaction medium (aqueous or organic) depends on a number of factors, the most important of which are the type of reaction, the stability of the catalytic system, the involvement of any co-factors which must be recycled, and the scale on which the biotransformation is to be carried out.

The main advantage of using whole cells is that it eliminates the need to recycle the co-factors required for the catalytic activity, because they are already present in the cell; the main drawback is the possible production of by-products, due to the action of enzymes other than those directly involved in the biotransformation or to the existence of a number of enzymes active on the same substrate, leading to a reduction in selectivity.

Many biological methods designed to produce compound (I) in optically active forms involve resolution of the racemic mixture obtainable by reacting glycerol with acetone, these substrates being widely and cheaply available (scheme 3).

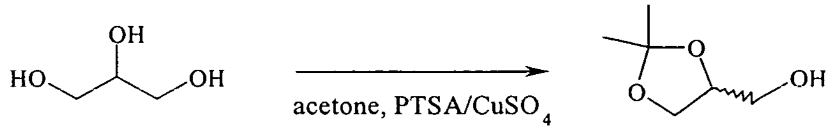


**Scheme 3**

A first method, described by International ByoSynthetics (Liese A., Seelbach K., Wandrey C., *Industrial Biotrasformations, 200* Wiley-vch pg 163) and illustrated in scheme 4, produces the R form and involves enantioselective oxidation of the S enantiomer, using *Rhodocococcus erythropolis* oxidase.

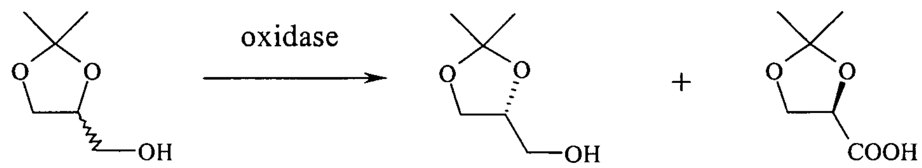


(R/S)-IPG (R)-IPG isopropylidene glyceric acid (R)

**Scheme 4**

The enantiomeric excess of the R form, with a molar conversion of 50%, proved to be over 98%.

A second method of resolution involves enantioselective acylation of the racemic mixture of alcohol by enzymes such as lipase or esterase in organic solvent.

Lipase AK of *Pseudomonas sp*, for example, catalyses the acylation reaction, but with limited stereoselectivity (enantiomeric ratio E = 5-30, depending on the acyl group) (Vänttinen E., Kanerva L.T. *Tetrahedron: Asymmetry.* 1996; 7: 3037), while other lipases originating from different microbial sources (*Rhizopus delemar, Rhizopus oryzae, Pseudomonas cepacia*, etc.) have given modest results (Okazaki S., Kamiya N., Goto M., Nakaschio F., *Biotecno. Lett.* 1997; 19:541).

A good alternative to the use of isolated enzymes is to employ whole freeze-dried cells suspended in organic solvent, as the cell structure is a natural matrix that protects enzymes against the action of external agents which can affect their activity.

Esterification studies (Laudadio L., "Ottenimento di derivati asimmetrici del glicerolo per via microbica", Degree dissertation in Food Science and Technology, Faculty of Agriculture, Academic Year 1997-1998, Milan) with butyric acid in heptane with freeze-dried micro-organisms such as *Yarrowia lipolytica, Aspergillus niger, Rhizopus delemar* and *Rhizopus oryzae*, have given ester yields of between 25 and 100%. However, the enantioselectivity found for conversions below 50% is rather limited (E < 5).

In a recent degree dissertation (Boni P., "Biotrasformazioni in solvente organico per la produzione di molecole di interesse agroalimentare", Degree dissertation in Plant Biotechnologies, Faculty of Agriculture, Academic Year 2000-2001, Milan) it was reported that different strains of *Rhizopus oryzae* catalyse the esterification of (I) with butyric acid in heptane at high speeds and with good enantioselectivity, but only in the first few hours of transformation, ie. at conversion values of under 40%. The best result obtained was an enantiomeric excess of 83% after one hour, with a molar conversion of 11 %.

A third method (scheme 5) involves transforming the racemic mixture of alcohol into that of the corresponding ester by conventional non-enantioselective methods and enantioselectively hydrolysing the esters by means of a hydrolase (lipase or esterase) or a whole cell (Wang Y-F., Lalonde J.J., Momongan M., Bergbraeter D.E., Wong C-H., 1998. *J. Am. Chem. Soc* 110, 7200; Phung A.N., Braun J., Le Goffic F., 1995. *Synthetic Communications* 25, 1983-1988; Aragozzini F., Maconi E., Scolastico S. 1991 Appl. Microbiol. Biotechnol. 34, 177-186).

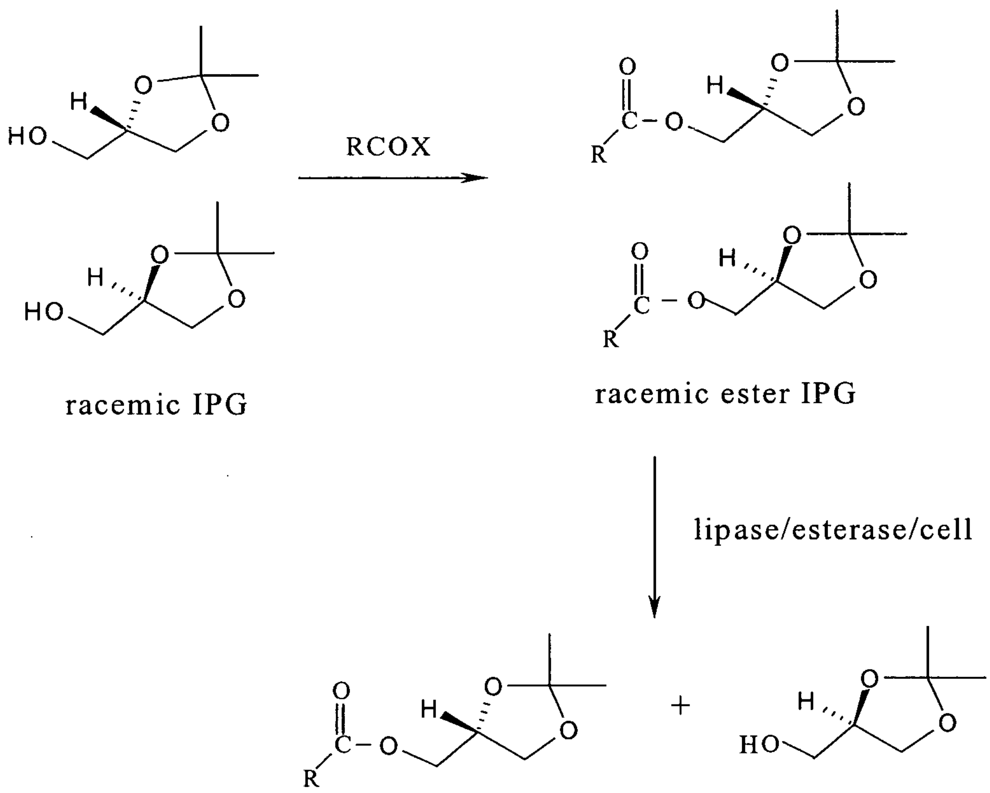


**Scheme 5**

As regards benzoic esters, for example, while hydrolysis catalysed by commercial enzymes has not produced satisfactory results, good results have been obtained using a combination of enzymatic hydrolysis and preferential crystallisation (Bianchi D., Bosetti A., Golini P., Pina C., *Tetrahedron: Asymmetry.* 1997; 8: 817), as illustrated in scheme 6.

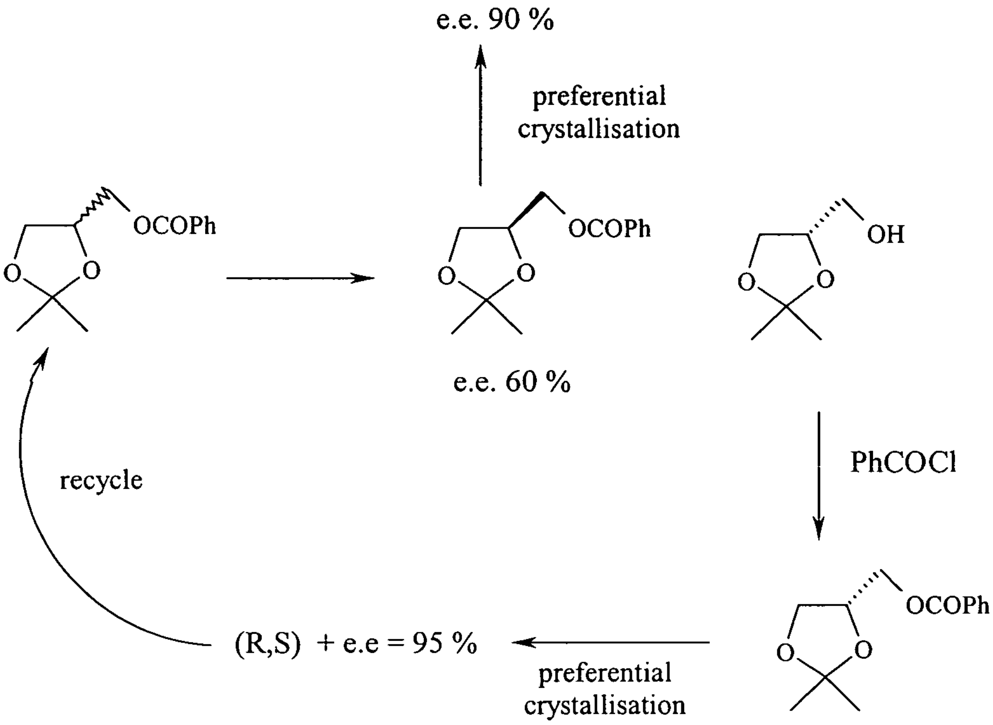


**Scheme 6**

Alternatively, hydrolysis can be performed with *Pseudomonas cepacia* lipase, using co-solvents able to influence the stereochemistry of the reaction (Bosetti A., Bianchi D., Cesti P., Golini P., *Biocatalysis.* 1994, 9:71).

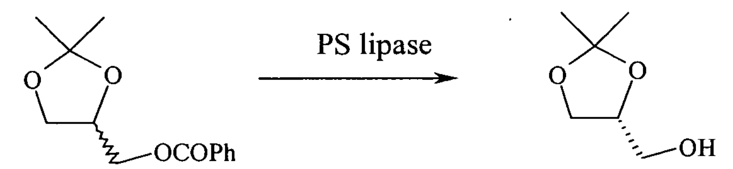


(R,S)-IPG benzoate (S)-IPG

An increase in the polarity of the solvent leads to a gradual increase in the enantiomeric excess (hereinafter called "e.e.") of the residual ester (from 67 for n-octane to 97 for dimethyl sulfoxide).

A strain of *Bacillus coagulans* able to hydrolyse racemic benzoic esters of IPG with an enantioselectivity greater than that of commercial enzymes (84% e.e. of S alcohol) has been identified on the basis of screening tests carried out on whole microbial cells of different genuses and species (Aragozzini F., Maconi E., Scolastico S. *Appl. Microbiol. Biotecnol.* 1991; 34: 433).

Hydrolysis tests on 1,2-O-isopropylidene glycerol acetate gave (R)-1,2-O-isopropylidene glycerol with modest results only when cell cultures of *Kluyveromyces marxianus* CBS 397 or *Saccharomyces cerevisiae* (e.e. of 56 and 52% respectively) were used. In this study, the racemic substrate was added directly to broth cultures, which also contained any extracellular enzymes.

It has now been found that enzymatic hydrolysis of the acetate with *Kluyveromyces marxianus* CBS 397 can be conveniently exploited to obtain (S)-1,2-isopropylidene glycerol with a high enantiomeric excess and satisfactory yields.

## DETAILED DESCRIPTION OF THE INVENTION

This invention relates to a process for the synthesis of (S)-1,2-O-isopropylidene glycerol

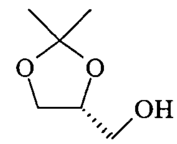

S-(I)

involving:

a. enzymatic hydrolysis of a racemic mixture of (R,S)-1,2-O-isopropylidene glycerol acetate (II), catalysed by *Kluyveromyces marxianus* or an extract or enzymatically active preparation thereof

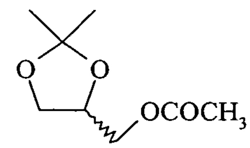

(II)

b. recovery of the R form of unreacted 1,2-O-isopropylidene glycerol acetate (II) and hydrolysis of the ester.

"Enzymatically active extract or preparation" means a cell fraction or extract which maintains the hydrolysis activity of the substrate.

A strain of *Kluyveromyces marxianus*, preferably selected from the strains deposited at the Centraalbureau voor Shimmelcultures under numbers CBS 397, CBS 834, CBS 2762, CBS 607, CBS 4857, CBS 1553, CBS 2231, and more preferably CBS 397, is used.

The enzymatic hydrolysis reaction takes place in an aqueous environment at a pH of between 6 and 8, preferably in a pH 7 phosphate buffer at a concentration of between 0.05 and 0.1 M, more preferably 0.067 M (hereinafter called "1/15 M"), at a temperature of between 27 and 30°C, optionally in the presence of a water-soluble co-solvent selected from acetone, ethanol and dimethyl sulfoxide, more preferably dimethyl sulfoxide, at a concentration of between 5 and 10 v/v. A yeast cell concentration of between 15 and 25 g/L and a 1,2-O-isopropylidene glycerol (II) concentration of between 1.5 and 30 g/L, preferably between 1.5 and 5 g/L, and even more preferably between 1 and 3 g/L, is used.

Part of the enzymatic activity is released by the cells into the aqueous solution used for the transformation; this takes place by simple prolonged suspension of the microbial cells in water or by mild agitation.

According to a preferred embodiment of the invention, the reaction is carried out semi-continuously, using a membrane bioreactor consisting of a reaction cell equipped with an agitation system and an ultrafiltration membrane with a cutoff of 10000 Da. After six hour, ie. when the enantiomeric excess is 100%, ultrafiltration is performed, so that the yeast cells and enzymes released remain in the reactor. More IPG acetate is added, and the reaction is recommenced. 5-10 reaction cycles are preferably performed, and more preferably 8 cycles. The aqueous phase is filtered and extracted with hexane or heptane, preferably hexane, and the (R)-1,2-O-isopropylidene glycerol acetate is purified by chromatography.

The successive hydrolysis of the ester is performed by conventional methods of hydrolysis of esters of 1,2-O-isopropylidene glycerol.

This invention is particularly advantageous compared with the method involving hydrolysis of the mixture of benzoic esters, because:

- fermentation of *Kluyveromyces marxianus* is simpler than that of *Bacillus coagulans*;
- *Kluyveromyces marxianus* grows on low-cost media such as whey or malt extract;
- 1,2-O-isopropylidene glycerol acetate is easy to synthesise and hydrolyse;
- the raw materials used to produce 1,2-O-isopropylidene glycerol acetate (glycerol, acetone and acetic acid) are all cheap.

The invention will now be illustrated in greater detail in the experimental section below.

## EXPERIMENTAL PART

### Materials and methods

The *Kluyveromyces marxianus* strains were obtained from the Centraalbureau voor Shimmelcultures (CBS), Baarn (NL), and are indicated by the following codes: CBS 397, 834, 2235, 2762, 607, 1553, 4857, 2231.

#### Maintenance conditions

The yeasts were kept viable by means of monthly transplants onto agar-malt (infusion of malt and agar = 15 g/l pH = 5.6, sterilisation $^3/_4$ atm for 20 minutes) with incubation at 28°C for 48 hours.

#### Submerged cultures

10 ml portions of cell suspension, obtained by resuspending in sterile water the cell patina of a solid culture obtainable in Petri dishes or in agar-malt slant tubes (after 24 h at 28°C), were inoculated into 100 ml of liquid malt-based medium (malt extract 100 g/l, spring water pH=5.6, sterilisation ¾ atm for 20 minutes) contained in 750 ml conical flasks. The flasks were placed on a linear agitator (120 spm) in a chamber thermostated at 28°C or 30°C for 2 or 48 hours.

Cells deriving from the submerged cultures were centrifuged at 8000 rpm for 10 minutes, washed with pH 7 phosphate buffer, and used under various conditions. The cells thus obtained are hereinafter called "fresh cells".

#### Freeze-drying

After cultivation in liquid medium, the yeast cells were separated from the culture broth by centrifugation at 8000 rpm for 10 minutes, using a Cavallo mod. 3228 centrifuge.

Two washes were then performed with 1/15 M phosphate buffer, pH = 7, and the cell base was suspended in a minimal volume of distilled water in a crystalliser.

The suspensions thus obtained were frozen at the temperature of -20°C and freeze-dried with an Edwards Minifast mdf 01 freeze dryer at the plate temperature of 25°C.

#### Synthesis of 1,2-O-isopropylidene glycerol acetate

30 ml of anhydrous benzene was placed in a 250 ml flask, and 4 g of (R,S)-1,2-O-isopropylidene glycerol and 8 ml of pyridine were added under magnetic agitation. 6 ml of distilled acetic anhydride was added, drop by drop, to the flask, immersed in an ice bath.

After 24 hours at room temperature, 100 ml of a 5% solution of sodium bicarbonate was slowly added to the reaction mixture under stirring; the organic phase was then separated from the aqueous phase with a separatory funnel.

Two extractions were then performed with 50 ml of ethyl acetate, the organic phases (benzene and ethyl acetate) were collected in a single conical flask, and the residual aqueous phase was discarded. The organic phase thus obtained was dried over anhydrous sodium sulphate, and the solvent was evaporated at 40°C under vacuum with a rotary evaporator. The crude residue was then purified by flash chromatography on a silica gel column pre-treated with a 1% solution of triethylamine, using hexane:ethyl acetate 1:1 as eluent.

The acetic ester thus obtained proved pure on thin-layer chromatography analysis (Merck Kieselgel. Eluent: hexane:ethyl acetate 1:1. Developer: oxidising mixture based on cerium and molybdenum).

#### Hydrolysis tests in phosphate buffer

The hydrolysis tests were performed by suspending 30 g/l of freeze-dried cells in 1/15 M phosphate buffer at pH 7. The reaction was carried out in 10 ml test tubes with screw cap and magnetic agitator, placed in a water bath at the temperature of 30°C.

IPG acetate was added at the concentration of 2.5 g/l.

#### Hydrolysis tests on IPG acetate with fresh *Kluyveromyces marxianus CBS 397* cells

Cells deriving from the submerged cultures were centrifuged at 8000 rpm for 10 minutes, washed with pH 7 phosphate buffer and used under different conditions.

Hydrolysis tests at different temperature, pH, cell concentration and substrate concentration values and with the use of co-solvents

The tests were carried out with fresh cells and freeze-dried cells.

In the tests carried out at different temperatures, 30 g/l of cells was suspended in 2.5 ml of 1/15 M phosphate buffer at pH 7, with an IPG acetate concentration of 2.5 g/l. The reaction was carried out at different temperatures: 27°C, 30°C, 35°C, 40°C and 50°C.

In the tests at different pH values, 30 g/l of cells were suspended in a volume of 2.5 ml of 1/15 M phosphate buffer at different pH values, in particular 6, 6.5, 7, 7.5 and 8.

IPG acetate was added at a concentration of 2.5 g/l, with a reaction temperature of 30°C.

In the tests at different cell concentrations, cell concentrations of between 15 and 35 g/l were suspended in 2.5 ml of 1/15 M phosphate buffer pH 7. The reaction was carried out at the temperature of 30°C with 2.5 g/l of IPG acetate.

In the tests at different substrate concentrations, 30 g/l of cells were suspended in 2.5 ml of phosphate buffer 1/15 M at pH 7, and IPG acetate concentrations of between 1.5 and 30 g/l were added. The reaction temperature was 30°C.

In the tests with co-solvents, 30 g/l of cells were suspended in 2.5 ml of phosphate buffer solution at pH 7 and water-soluble solvent at a concentration of between 5 and 10 v/v.

The solvents used were acetone, dimethyl sulfoxide, dimethylformamide and ethanol. The reaction was carried out at the temperature of 30°C, by adding 2.5 g/l of IPG acetate.

Heat pre-treatment tests

Yeast cells suspended in 2.5 ml of 1/15 M phosphate buffer at pH 7 at the concentration of 30 g/l were maintained under agitation at different temperatures (40°C, 45°C and 50°C) for 30 minutes (and for 60 minutes at 40°C). The reaction was then initiated at the temperature of 30°C by adding 2.5 g/l of IPG acetate.

Yeast cell recycling tests

After a 24-hours reaction (T = 30°C, [S] = 2.5 g/l, [cell.] = 30/l, in pH 7 phosphate buffer), the yeast cells were filtered through a Buchner funnel and washed twice with 1/15 M phosphate buffer pH 7. The reaction was then performed again under the previous conditions.

Evaluation of enzymatic release in the reaction medium, and effect of acetic acid

The release of enzymatic activity in the reaction medium was evaluated by maintaining the cells (in 1/15 M, pH = 7 phosphate buffer at 30°C at the concentration of 30 g/l) under agitation for different times, and then adding IPG acetate at the concentration of 2.5 g/l. After a 24-hours reaction the cells were separated from the reaction medium by centrifugation at 4000 rpm for 15 minutes. Hydrolysis tests were then carried out again under the previous conditions, using the cell residue and the supernatant obtained.

The effect of acetic acid was evaluated by tests in which varying amounts of acetic acid (in a molar ratio of 0.5, 1 and 2) were added to the reaction mixture, operating under the previous conditions.

Hydrolysis tests with immobilised cells

The yeast cells were immobilised in calcium and barium alginate matrixes.

The immobilisation was performed by dropping 10 ml of a 30 g/l cell suspension in alginic acid (as 2% sodium salt) into a 40 ml solution of 0.1 M calcium (or barium) chloride, under stirring.

The spheres thus obtained were filtered through a Buchner funnel, washed with Tris-HCl buffer at pH 7 and returned to an conical flask containing 15 ml of 0.1 M Tris-HCl buffer at pH 7. The reactions were carried out at the temperature of 30°C by adding 2.5 g/l of IPG acetate under alternating agitation.

The recycling tests were carried out after 24 hours' reaction, the alginate spheres being filtered and washed with pH 7 Tris-HCl buffer; the hydrolysis reaction was performed under the same conditions as the previous tests.

Use of a membrane bioreactor

An AMICON model 8050 ultrafiltration cell equipped with a magnetic agitation system, and a Millipore ultrafiltration membrane with a cut-off of 10000, were used.

The reaction was carried out with a 30 g/l suspension of yeast cells in 50 ml of 1/15 M phosphate buffer at pH 7, at an IPG acetate concentration of 2.5 g/l and a temperature of 30°C.

After approximately 6 hours, ie. the time when the e.e. of the IPG acetate reached 100%, 40 ml of the reaction mixture was subjected to ultrafiltration by $N_2$ flow at a pressure of 2.5 bars, with a filtration flow of ~ 2 ml/min.

After the ultrafiltration, 40 ml of fresh buffer was added to 10 ml of residues, and the reaction was recommenced by adding 2.5 g/l of IPG acetate.

After 5 reaction cycles the filtrates were collected (~ 200 ml) and three extractions were performed with 50 ml of hexane. After anhydrising with anhydrous sodium sulphate, the hexane was evaporated under vacuum at 40°C with a rotavapor.

The crude residue was then purified by flash chromatography on a silica gel column pre-treated with a 1% solution of triethylamine, using hexane:ethyl acetate 1:1 as eluent.

The IPG acetate thus obtained proved enantiomerically pure on gas chromatography analysis.

## Analysis methods

### Quantitation

The quantitation over time of the hydrolysis reaction of 1,2-O-isopropylidene glycerol acetate was performed by gas chromatography, using the internal standard method.

The trend of the reaction was evaluated by sampling 100 µl of the reaction mixture at defined time intervals, extracting with 100 µl of ethyl acetate containing the internal standard, and injecting 1 µl into the column.

A Carlo Erba Fractovap series 2150 gas chromatograph, equipped with a flame-ionisation detector and fitted with a 4 × 1500 mm glass column packed with 10% Carbowax 20 M on 100/120 mesh Supelcoport, was used under the following operating conditions:

- ° Carrier pressure ($N_2$): 1 Kg / $cm^2$
- ° Air pressure: 1.5 Kg / $cm^2$
- ° Hydrogen pressure: 0.8 Kg / $cm^2$

The internal standard was n-octanol at the concentration of 1 g/l.

The gas chromatography conditions are set out below:

Table 1

| Compound | Column Temperature * (°C) | Retention times (minutes) |
|---|---|---|
| IPG | 130 | 10.26 |
| | 140 | 9.2 |
| IPG acetate | 130 | 13.7 |
| 1-octanol | 130 | 12.9 |
| | 140 | 10.7 |
| Gas chromatography conditions for quantitation of the components of the reaction mixture. | | |

* Carbovax 20 M column

### Evaluation of enantiomeric excess

The enantiomeric excess was calculated with the formula:

$$e.e.\ (\%) = \frac{\text{majority enantiomer - minority enantiomer}}{\text{majority enantiomer + minority enantiomer}} \times 100$$

The relative quantities of each enantiomer, expressed in terms of areas, were determined with a Dani 6500 gas chromatograph, equipped with flame-ionisation detector, to which a 25-metre dimethylpentyl beta capillary column, CDX 30% and PS 70% (Mega, Legnano), was fitted.

The chromatographic separation was performed by operating with the following temperature gradients:

Table 2

| Compound | Temperature gradient (°C) | Retention times (minutes) | |
|---|---|---|---|
| | | R | S |
| IPG acetate | 90°C for 10 min., 2°C/min. to 120°C for 10 min | 13.4 | 12.8 |
| IPG | 90°C for 10 min., 2°C/min. to 120°C for 10 min | 8.5 | 9.2 |
| | 90°C for 10 min., 4°C/min. to 110°C for 10 min | 9.1 | 9.8 |

Gas chromatography conditions for the determination of single enantiomers.

A pure gas chromatography standard of (S)-1,2-O-isopropylidene glycerol was used to identify the enantiomer.

## Results

On the basis of screening studies carried out on micro-organisms of different genuses and species (yeasts of the genus *Saccharomyces*, *Kluyveromyces* and *Rhodotorula,* moulds of the genus *Rhizopus* and the bacterium *Micrococcus luteus*) with hydrolysis tests carried out on 1,2-O-isopropylidene glycerol acetate at the concentration of 2.5 g/L in 1/15 M phosphate buffer at pH 7, at the temperature of 30°C, it was observed that all the micro-organisms catalyse the hydrolysis of both esters of 1,2-O-isopropylidene glycerol, except for *Kluyveromyces marxianus* CBS 397, which gave an enantiomeric excess of (R)-1,2-O-isopropylidene glycerol acetate of approx. 15 (Figure 1).

The hydrolysis reaction, at a temperature of 30°C, an IPG acetate concentration of 2.5 g/l and a cell concentration of 30 g/l in 1/15 M phosphate buffer at pH 7, takes place with either freeze-dried or fresh cells. No significant differences in reaction rate or enantioselectivity were observed. With fresh cells the enantioselectivity declines slightly, while the reaction rate increases (Table 3).

Table 3

| Time (hours) | FRESH CELLS | | | | FREEZE-DRIED CELLS | | | |
|---|---|---|---|---|---|---|---|---|
| | e.e. % (R) IPG acetate | e.e.% IPG | Molar conv. (%) | E | e.e. % (R) IPG acetate | e.e. % (R) IPG | Molar conv. (%) | E |
| 1 | 53 | 78 | 40 | 14 | 45 | 82 | 35 | 15 |
| 3 | 91 | 67 | 58 | | 84 | 71 | 54 | |
| 5 | 98 | 54 | 64 | | 94 | 65 | 59 | |

Comparison between fresh and freeze-dried cells for the hydrolysis of 2.5 g/l of IPG acetate, in 1/15 M phosphate buffer at pH 7, at the temperature of 30°C.

*Kluyveromyces marxianus* cells originating from submerged, centrifuged cultures can therefore be used. With these cells, the highest enantioselectivity is obtained by carrying out the reaction at a temperature of between 27 and 30°C, if the hydrolysis is performed in a 1/15 M phosphate buffer with an IPG acetate concentration of 2.5 g/L and a cell concentration of 30 g/L (Table 4).

Table 4

| Temperature (°C) | Time (hours) | e.e. % (R) IPG acetate | e.e. % (R) IPG | Molar conv. (%) | E |
|---|---|---|---|---|---|
| 27 | 3 | 85 | 70 | 55 | 15 |
| | 5 | 94 | 64 | 59 | |
| | 24 | > 98 | 43 | 70 | |

Table 4 (continued)

| Temperature (°C) | Time (hours) | e.e. % (R) IPG acetate | e.e. % (R) IPG | Molar conv. (%) | E |
|---|---|---|---|---|---|
| 30 | 3 | 84 | 71 | 54 | 15 |
| | 5 | 92 | 66 | 58 | |
| | 24 | > 98 | 40 | 70 | |
| 35 | 3 | 71 | 71 | 50 | 13 |
| | 5 | 84 | 64 | 57 | |
| | 24 | > 98 | 43 | 70 | |
| 40 | 3 | 88 | 65 | 58 | 12 |
| | 5 | 95 | 53 | 64 | |
| | 24 | > 98 | 38 | 72 | |
| 50 | 3 | 55 | 64 | 46 | 6 |
| | 5 | 60 | 43 | 58 | |
| | 24 | 64 | 30 | 68 | |
| Hydrolysis of IPG acetate catalysed by *Kluyveromyces marxianus* CBS 397, at different temperature values. | | | | | |

As regards the pH, it was observed that the highest enantioselectivity at the temperature of 30°C, under the same yeast and substrate concentration conditions, is obtained at pH values between 7 and 8 (Table 5).

Table 5

| pH | Time (hours) | e.e. % (R) IPG acetate | e.e. % (R) IPG | Molar conv. (%) | E |
|---|---|---|---|---|---|
| 6 | 3 | 40 | 63 | 39 | 6.5 |
| | 5 | 55 | 60 | 48 | |
| 6.5 | 1 | 33 | 79 | 29 | 10 |
| | 3 | 62 | 67 | 48 | |
| | 5 | 76 | 61 | 56 | |
| 7 | 1 | 45 | 82 | 35 | 15 |
| | 3 | 84 | 71 | 54 | |
| | 5 | 94 | 65 | 59 | |
| 7.5 | 1 | 80 | 56 | 41 | 15 |
| | 3 | 97 | 62 | 61 | |
| | 5 | > 98 | 49 | 67 | |
| 8 | 1 | 63 | 77 | 45 | 15 |
| | 3 | > 98 | 57 | 63 | |
| Hydrolysis of IPG-acetate catalysed by *Kluyveromyces marxianus* CBS 397, at different pH values. | | | | | |

The cell concentration was evaluated in a 1/15 M phosphate buffer at pH 7, at the temperature of 30°C and a substrate concentration of 2.5 g/L. Although the differences are fairly limited, it will be seen from the data reported in Table 6 that the best results are achieved at the highest cell concentration, ie. 30 to 35 g/l.

Table 6

| Cell Conc. (g/l) | Time (hours) | e.e. % (R) IPG acetate | e.e. % (R) IPG | Molar conv. (%) | E |
|---|---|---|---|---|---|
| 15 | 3 | 47 | 77 | 38 | 12 |
| | 5 | 65 | 74 | 47 | |
| | 24 | 92 | 61 | 60 | |
| 20 | 3 | 59 | 75 | 44 | 12 |
| | 5 | 76 | 70 | 52 | |
| | 24 | 95 | 46 | 67 | |
| 25 | 3 | 68 | 73 | 48 | 13 |
| | 5 | 83 | 70 | 54 | |
| | 24 | > 98 | 48 | 72 | |
| 30 | 3 | 84 | 71 | 54 | 15 |
| | 5 | 94 | 65 | 59 | |
| | 24 | 98 | 42 | 70 | |
| 35 | 3 | 81 | 72 | 53 | 15 |
| | 5 | 96 | 60 | 62 | |
| | 24 | > 98 | 41 | 71 | |
| Hydrolysis of IPG acetate catalysed by *Kluyveromyces marxianus* CBS 397, at different cell concentration values. | | | | | |

As enzymatic systems can be sensitive to high concentrations of artificial molecules, hydrolysis tests were carried out in a 1/15 M phosphate buffer at pH 7, with a cell concentration of 30 g/l, a temperature of 30°C and IPG acetate concentrations of between 1.5 and 30 g/l. The data reported in Table 7 demonstrate that hydrolysis takes place with significant conversion even as from 30 g/l (molar conversion of 31% after 5 h), although at concentrations exceeding 5 g/l a reduction in enantioselectivity was observed.

Table 7

| Conc. (g/l) IPG acetate | Time (hours) | e.e. % (R) IPG acetate | e.e. % (R) IPG | Molar conv. (%) | E |
|---|---|---|---|---|---|
| 1.5 | 3 | 69 | 77 | 47 | 15 |
| | 4 | 85 | 69 | 55 | |
| | 24 | > 98 | 45 | 69 | |
| 2 | 3 | 76 | 73 | 51 | 15 |
| | 5 | 90 | 69 | 57 | |
| | 24 | > 98 | 39 | 72 | |
| 2.5 | 3 | 80 | 71 | 53 | 15 |
| | 5 | 93 | 66 | 58 | |
| | 24 | > 98 | 39 | 73 | |
| 3 | 3 | 72 | 75 | 49 | 15 |
| | 5 | 87 | 70 | 57 | |
| | 24 | > 98 | 40 | 73 | |

Table 7 (continued)

| Conc. (g/l) IPG acetate | Time (hours) | e.e. % (R) IPG acetate | e.e. % (R) IPG | Molar conv. (%) | E |
|---|---|---|---|---|---|
| 5 | 3 | 67 | 73 | 48 | 12 |
| | 5 | 81 | 67 | 55 | |
| | 24 | > 98 | 43 | 70 | |
| 10 | 3 | 41 | 77 | 35 | 11 |
| | 5 | 60 | 73 | 45 | |
| | 24 | 90 | 56 | 62 | |
| 20 | 3 | 36 | 76 | 32 | 10 |
| | 5 | 40 | 75 | 35 | |
| | 24 | 64 | 66 | 49 | |
| 30 | 3 | 26 | 76 | 25 | 8 |
| | 5 | 32 | 70 | 31 | |
| | 24 | 45 | 68 | 39 | |
| Hydrolysis of IPG acetate catalysed by *Kluyveromyces marxianus* CBS 397, at different concentration values of the substrate, IPG acetate. | | | | | |

As variations in the composition of the reaction medium can lead to changes in enantioselectivity due to selective enzymatic inhibition, modification of the enzyme conformation or different solubility of the substrate, water-soluble polar solvents at different concentrations were added to the reaction medium (1/15 M phosphate buffer at pH 7). An increase in enantioselectivity was observed with ethanol, acetone and DMSO, probably due to inhibition of the less enantioselective enzymes (Table 8).

Table 8

| Co-solvent | v/v % | Time (hours) | e.e % (R) IPG acetate | e.e. % (R) IPG | Molar conv. (%) | E |
|---|---|---|---|---|---|---|
| acetone | 5 | 3 | 48 | 83 | 37 | 17 |
| | | 5 | 72 | 77 | 48 | |
| | 10 | 3 | 50 | 82 | 38 | 17 |
| | | 5 | 74 | 80 | 49 | |
| dimethylformamide | 5 | 3 | 29 | > 98 | 23 | 14 |
| | | 5 | 44 | 82 | 34 | |
| dimethyl sulfoxide | 5 | 3 | 87 | 78 | 53 | 23 |
| | | 5 | 97 | 66 | 60 | |
| | 10 | 3 | 82 | 78 | 51 | 23 |
| | | 5 | 97 | 70 | 58 | |
| ethanol | 5 | 3 | 78 | 78 | 50 | 18 |
| | | 5 | 94 | 67 | 58 | |
| | 10 | 3 | 50 | 81 | 38 | 18 |
| Hydrolysis of IPG acetate catalysed by *Kluyveromyces marxianus* CBS 397, in the presence of different solvents. | | | | | | |

For a preliminary evaluation of the stability of *Kluyveromyces marxianus* CBS 397 under the operating conditions used, tests were carried out wherein the cells used for hydrolysis after 24 hours were filtered, washed and prepared for new hydrolysis.

The cells showed a drastic reduction in activity, which can probably be ascribed to the low stability of the enzymes involved in the reaction conditions (T = 30°C, [S] = 2.5 g/l, [cell.] = 30 g/l) or to release of part of the enzyme content during washing of the whole cells (Table 9).

Table 9

| Time | e.e. % ( R ) IPG acetate | e.e. % (R) IPG | Molar coav. (%) | E |
|---|---|---|---|---|
| 3 | 7 | 62 | 16 | 3 |
| 5 | 16 | 44 | 27 | |
| 24 | 24 | 33 | 42 | |
| First recycling test on washed, filtered *Kluyveromyces marxianus* CBS 397 *cells*. | | | | |

This latter hypothesis was tested by maintaining the cells under agitation for different times and evaluating over time the residual activity associated with the cells or released. It was found that part of the hydrolytic activity is released into the supernatant and that if the cells are maintained under agitation, the enzymatic activity declines over time (Figures 2a and 2b).

The reduction in enzymatic activity is probably due to the acetic acid released into the reaction medium during hydrolysis. This inhibiting effect appears to be mainly exercised towards the most selective carboxylesterases. It has been found that by increasing the acetic acid in the reaction medium, the reaction rate declines slightly, and a definite reduction in enzymatic activity is observed (Table 10).

Table 10

| Molar ratio $\frac{\text{acetic acid added}}{\text{IPG}}$ | Time (hours) | e.e. % (R) IPG acetate | e.e. % (R) IPG | Molar conv. (%) | E |
|---|---|---|---|---|---|
| 0.5 | 1 | 35 | > 98 | 26 | 13 |
| | 3 | 80 | 67 | 54 | |
| | 24 | 98 | 55 | 64 | |
| 1 | 1 | 29 | > 98 | 23 | 10 |
| | 3 | 60 | 73 | 45 | |
| | 24 | 96 | 47 | 67 | |
| 2 | 1 | 13 | > 98 | 12 | 6 |
| | 3 | 27 | > 98 | 21 | |
| | 24 | 60 | 53 | 53 | |
| Hydrolysis tests on IPG acetate with the addition of different quantities of acetic acid. | | | | | |

To assess whether heat pre-treatment can deactivate the less enantioselective enzymes, hydrolysis tests were carried out at 30°C in a pH 7 phosphate buffer, with an IPG acetate concentration of 2.5 g/l, after maintaining the yeast cells (30 g/l) at different temperatures for 30 minutes (Table 11).

The best results were obtained by pre-treating the cells at 40°C, although no evident improvement was observed compared with the tests carried out without heat pre-treatment.

Table 11

| Temp. °C | Pre-treatment time (minutes) | Time (hours) | e.e.% (R) IPG acetate | e.e. % (R) IPG | Molar conv. (%) | E |
|---|---|---|---|---|---|---|
| 40 | 30 | 1 | 19 | > 98 | 16 | 16 |
| | | 3 | 56 | 80 | 41 | |
| | | 5 | 75 | 77 | 49 | |
| 40 | 60 | 1 | 18 | > 98 | 16 | 13 |
| | | 3 | 48 | 79 | 38 | |
| | | 5 | 68 | 70 | 49 | |
| 45 | 30 | 1 | 22 | > 98 | 18 | 10 |
| | | 3 | 59 | 67 | 47 | |
| | | 5 | 80 | 64 | 56 | |
| 50 | 30 | 1 | 10 | 78 | 11 | 8 |
| | | 3 | 18 | 75 | 19 | |
| Hydrolysis of IPG acetate with *Kluyveromyces marxianus* CBS 397 cells, pre-treated at different temperatures. | | | | | | |

A test was also carried out by pre-treating the cells at 40°C for 60 minutes, without obtaining an appreciable change in the activity of the catalyst.

As immobilisation can potentially improve the stability of the catalytic system, the cells were immobilised in calcium alginate or barium alginate to carry out hydrolysis tests on IPG acetate (Table 12). After a 24-hour reaction, the alginate spheres containing the cells were filtered and washed, the reaction was recommenced and the progress checked. However, immobilised cells give results similar to those of free suspended cells, and despite trapping, the washed, recycled cells release a certain quantity of enzyme.

Table 12

| Matrix | Time (hours) | e.e. % ( R ) IPG acetate | e.e. % (R) IPG | Molar conv. (%) | E |
|---|---|---|---|---|---|
| | 3 | 50 | 79 | 39 | |
| Ca alginate | 5 | 77 | 76 | 50 | 15 |
| | 24 | > 98 | 55 | 64 | |
| | 3 | 46 | 79 | 39 | |
| Ba alginate | 5 | 68 | 72 | 46 | 14 |
| | 24 | > 98 | 44 | 69 | |
| Ca alginate + 0.1M glutaraldehyde | 24 | - | - | - | - |
| Ca alginate + 0.5M glutaraldehyde | 24 | - | - | - | - |
| Hydrolysis tests on IPG acetate with immobilised *Kluyveromyces marxianus* CBS 397 cells. | | | | | |

Hydrolysis tests were carried out on IPG acetate with resuspended cells of other strains of *Kluyveromyces marxianus*, operating at the temperature of 30°C, [S] = 2.5 g/l, [cell.] = 30 g/l, in 1/15 M phosphate buffer at pH 7 (Table 13).

Table 13

| Strain | Time (hours) | e.e. % (R) IPG acetate | e.e. % (R) IPG | Molar conv. (%) | E |
|---|---|---|---|---|---|
| CBS 397 | 1 | 45 | 82 | 35 | 15 |
| | 3 | 84 | 71 | 54 | |
| | 5 | 94 | 65 | 59 | |
| CBS 834 | 1 | 68 | 74 | 48 | 15 |
| | 3 | > 98 | 53 | 65 | |
| CBS 2235 | 5 | - | - | - | - |
| CBS 2762 | 1 | 58 | 77 | 43 | 13 |
| | 3 | 96 | 56 | 65 | |
| CBS 607 | 1 | 53 | 76 | 41 | 12 |
| | 3 | 81 | 68 | 54 | |
| | 5 | 90 | 57 | 61 | |
| CBS 4857 | 1 | 51 | 81 | 39 | 15 |
| | 3 | 90 | 66 | 58 | |
| | 5 | > 98 | 54 | 65 | |
| CBS 1553 | 1 | 90 | 69 | 57 | 16 |
| | 3 | > 98 | 54 | 65 | |
| CBS 2231 | 1 | 55 | 78 | 41 | 13 |
| | 3 | 96 | 58 | 62 | |
| | 5 | > 98 | 50 | 66 | |

Hydrolysis of acetic ester of IPG catalysed by different strains of *Kluyveromyces marxianus*.

The results demonstrate that all the strains used, apart from strain CBS 2235, show enantioselective enzymatic activity similar to that of strain CBS 397.

Heat pre-treatment tests were carried out to test for the presence of several enzymes able to hydrolyse IPG-acetate with different enantioselectivity, and to evaluate the effect on the overall enantioselectivity of the whole cells (Table 14).

Hydrolysis tests were thus performed with different strains, the cells being pre-treated at 40°C for 1 hour.

Table 14

| Strain | Time | e.e. % (R) IPG acetate | e.e. % (R) IPG | Molar conv. (%) | E |
|---|---|---|---|---|---|
| CBS 397 | 1 | 18 | 96 | 16 | 13 |
| | 3 | 48 | 79 | 38 | |
| | 5 | 69 | 70 | 49 | |
| CBS 834 | 1 | 38 | 79 | 32 | 16 |
| | 3 | 88 | 71 | 55 | |
| | 5 | 97 | 49 | 66 | |

Table 14 (continued)

| Strain | Time | e.e. % (R) IPG acetate | e.e. % (R) IPG | Molar conv. (%) | E |
|---|---|---|---|---|---|
| CBS 2762 | 1 | 39 | 76 | 34 | 13 |
| | 3 | 88 | 70 | 56 | |
| | 5 | 91 | 53 | 63 | |
| CBS 607 | 1 | 12 | > 98 | 11 | 6 |
| | 3 | 21 | 67 | 24 | |
| CBS 1553 | 1 | 72 | 79 | 48 | 18 |
| | 3 | > 98 | 59 | 63 | |
| CBS 4857 | 1 | 36 | 81 | 31 | 13 |
| | 3 | > 98 | 49 | 67 | |
| CBS 2231 | 3 | 83 | 70 | 54 | 14 |
| | 5 | 97 | 59 | 61 | |
| Hydrolysis of IPG acetate with different strains of *Kluyveromyces marxianus* cells, pre-treated at 40°C for 1 hour. | | | | | |

The results shown in the table demonstrate that a considerable improvement in enantioselectivity was only achieved in the case of strain CBS 1553; in the other cases the opposite effect was observed, with a reduction in the enantiomeric ratio.

Synthesis of optically pure IPG using a membrane reactor

A membrane reactor was prepared, consisting of a filtration cell equipped with an agitation system and an ultrafiltration membrane with cut-off of 10000 D, so as to keep the released enzymes and cells in the reactor (Figure 3). The volume used was 100 ml.

The biotransformation was performed directly in the ultrafiltration cell, and after 5-6 hours, when the e.e. of the IPG acetate was 100%, ultrafiltration was performed to keep the cells and enzymes released in the reactor, and at the same time filter the aqueous phase containing the optically pure IPG acetate and the IPG deriving from hydrolysis.

This operation was performed for 8 successive cycles, and no reduction in the catalytic efficiency of the system was observed. It was thus possible to obtain 6.9-7.0 g/l of optically pure IPG acetate (analytical evaluation). By means of a series of extractions with hexane from the aqueous phase and purification with a chromatography column, 630 mg of optically pure IPG acetate was isolated.

## Claims

**1.** Process for the synthesis of (S)-1,2-O-isopropylidene glycerol (I)

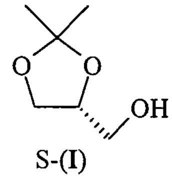

S-(I)

comprising the following steps:

a. enzymatic hydrolysis of a racemic mixture of (R,S)-1,2-O-isopropylidene glycerol acetate (II)

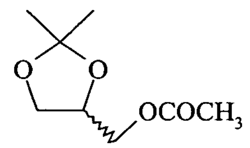

**(II)**

catalysed by *Kluyveromyces marxianus* or an extract or enzymatically active preparation thereof;
b. recovery of the R form of unreacted 1,2-O-isopropylidene glycerol acetate (II) and hydrolysis of the ester.

**2.** Process as claimed in claim 1, using a strain of *Kluyveromyces marxianus* selected from the strains deposited at the Centraalbureau voor Shimmelcultures under numbers CBS 397, CBS 834, CBS 2762, CBS 607, CBS 4857, CBS 1553 and CBS 2231.

**3.** Process as claimed in claim 2, using *Kluyveromyces marxianus* CBS 397.

**4.** Process as claimed in any one of claims 1-3, wherein the enzymatic hydrolysis is carried out in an aqueous environment at a pH of between 6 and 8.

**5.** Process as claimed in claim 4, wherein the enzymatic hydrolysis takes place in a phosphate buffer at pH 7.

**6.** Process as claimed in any one of claims 1-5, wherein the enzymatic hydrolysis is carried out at a temperature of between 27 and 30°C.

**7.** Process as claimed in any one of claims 1-6, wherein a *Kluyveromyces marxianus* cell concentration of between 15 and 25 g/L and a 1,2-O-isopropylidene glycerol acetate concentration of between 1.5 and 30 g/L are used.

**8.** Process as claimed in claim 7, wherein the concentration of 1,2-O-isopropylidene glycerol acetate is between 1.5 and 5 g/L.

**9.** Process as claimed in claim 8, wherein the concentration of 1,2-O-isopropylidene glycerol acetate is between 1.5 and 3 g/L.

**10.** Process as claimed in any one of claims 1-9, wherein a water-soluble co-solvent is used at a concentration of between 5 and 10 v/v compared with water.

**11.** Process as claimed in claim 10, wherein the water-soluble co-solvent is selected from acetone, ethanol and dimethyl sulfoxide.

**12.** Process as claimed in claim 11, wherein the co-solvent is dimethyl sulfoxide.

**Fig. 1**

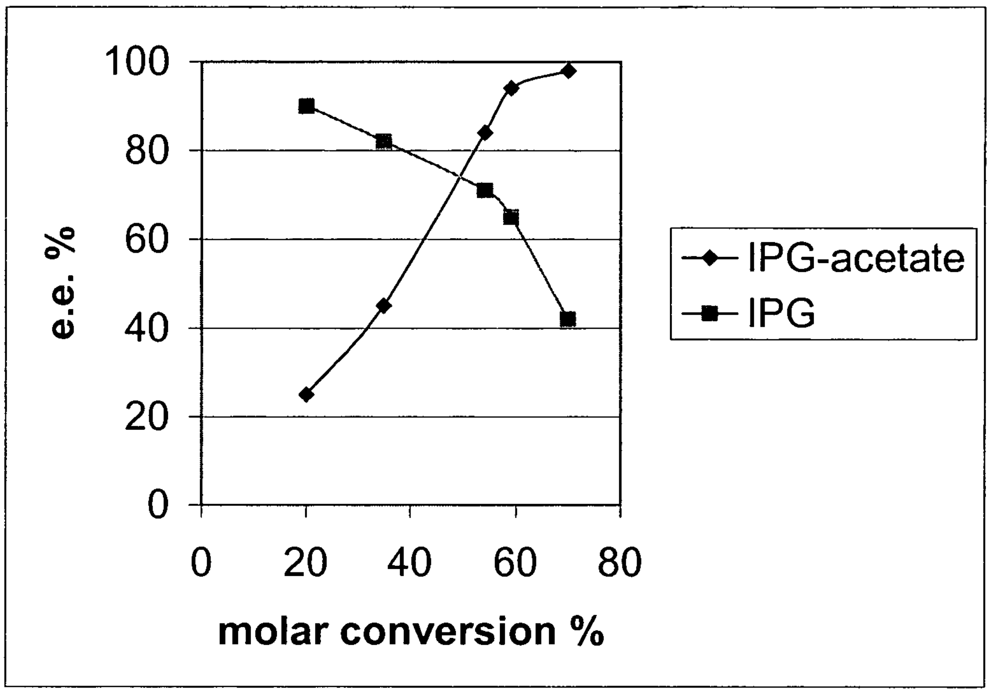


**Stereochemical trend of hydrolysis of IPG acetate catalysed by freeze-dried *Kluyveromyces marxianus* CBS 397 cells in pH 7 phosphate buffer.**

Fig. 2a

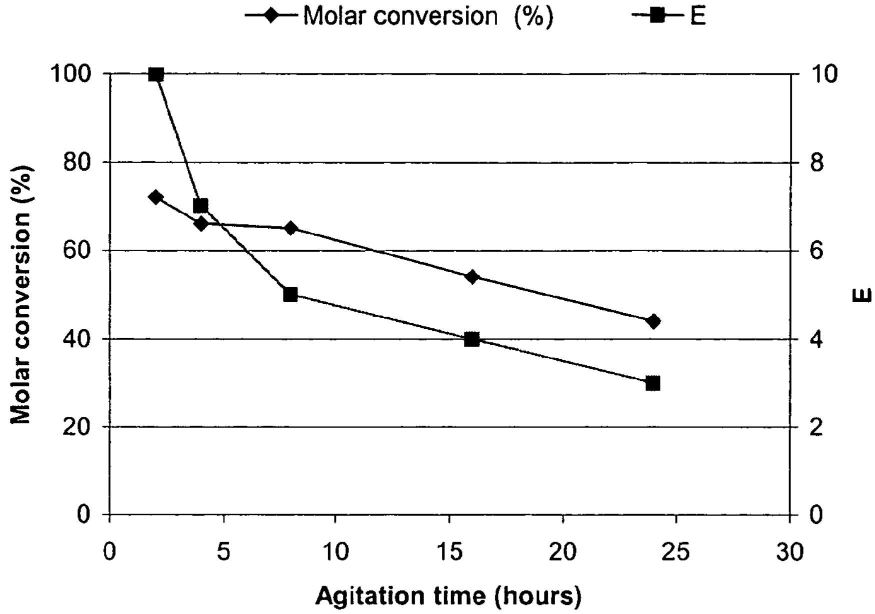


**Hydrolytic activity of the cell residue after different agitation times. The data are reported 24 hours after the addition of IPG acetate.**

Fig. 2b

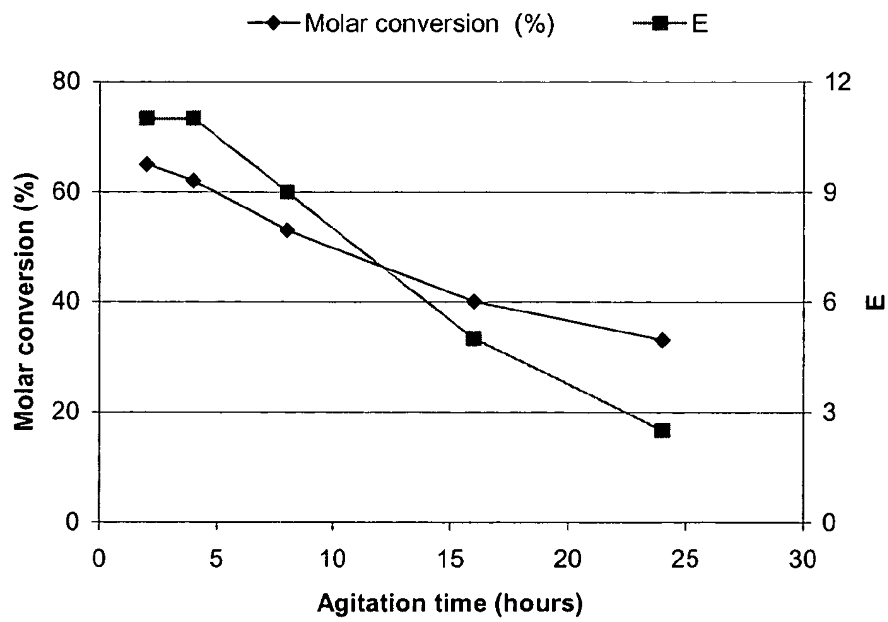


**Hydrolytic activity of the supernatant after different agitation times. The data are reported 24 hours after the addition of IPG acetate.**

Fig. 3

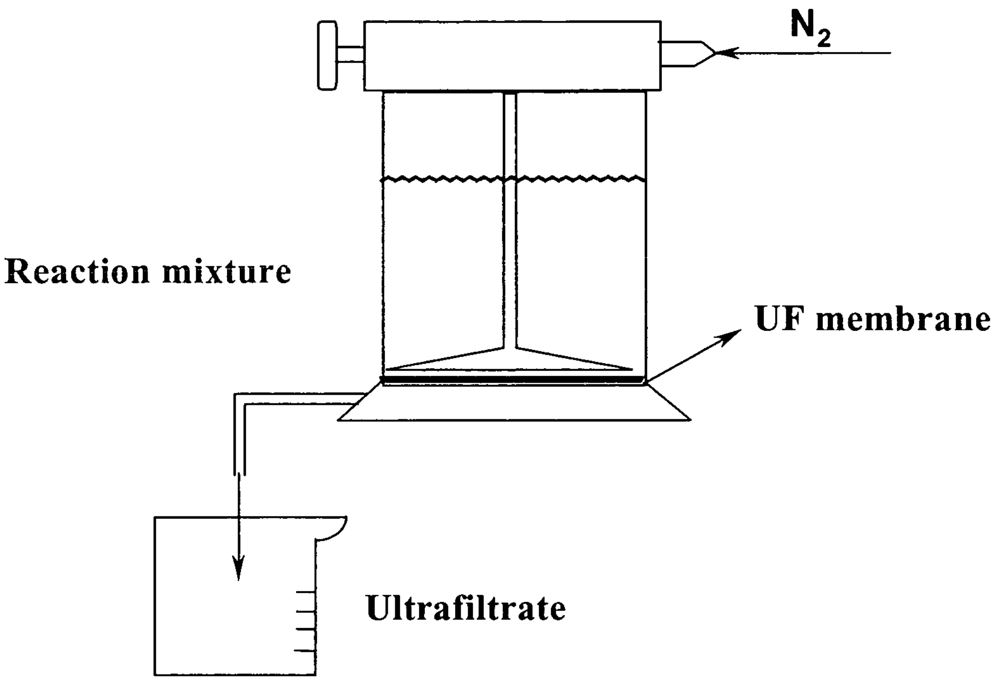

European Patent Office

# EUROPEAN SEARCH REPORT

Application Number
EP 03 02 4203

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | DROGE MELLONEY J ET AL: "Paralogous gene analysis reveals a highly enantioselective 1,2-O-isopropylideneglycerol caprylate esterase of Bacillus subtilis" EUROPEAN JOURNAL OF BIOCHEMISTRY, vol. 268, no. 11, June 2001 (2001-06), pages 3332-3338, XP002265375 ISSN: 0014-2956 * page 3336; figure 3 * --- | 1-12 | C12P17/04 C12P41/00 |
| A | EP 0 556 909 A (MINI RICERCA SCIENT TECNOLOG) 25 August 1993 (1993-08-25) * the whole document * --- | 1-12 | |
| A | PARTALI V ET AL: "ENZYMATIC RESOLUTION OF 2,2-DISUBSTITUTED-1,3-DIOXOLANE-4-METHANOL CARBOXYLIC ESTERS" TETRAHEDRON: ASYMMETRY, ELSEVIER SCIENCE PUBLISHERS, AMSTERDAM, NL, vol. 3, no. 1, 1992, pages 65-72, XP001026000 ISSN: 0957-4166 * page 66; table 2 * --- | 1-12 | TECHNICAL FIELDS SEARCHED (Int.Cl.7) C12P |
| A | VANTTINEN E ET AL: "Optimized double kinetic resolution for the preparation of (S)-solketal" TETRAHEDRON: ASYMMETRY, ELSEVIER SCIENCE PUBLISHERS, AMSTERDAM, NL, vol. 8, no. 6, 27 March 1997 (1997-03-27), pages 923-933, XP004094364 ISSN: 0957-4166 * the whole document * --- -/-- | 1-12 | |

The present search report has been drawn up for all claims

| Place of search | Date of completion of the search | Examiner |
|---|---|---|
| MUNICH | 18 December 2003 | Pinheiro Vieira, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 03 02 4203

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | SUEMUNE H ET AL: "ENZYMATIC SYNTHESIS OF 2,3-O-ISOPROPYLIDENE-SN-GLYCEROL, A CHIRAL BUILDING BLOCK FOR PLATELET-ACTIVATING FACTOR" CHEMICAL AND PHARMACEUTICAL BULLETIN, PHARMACEUTICAL SOCIETY OF JAPAN. TOKYO, JP, vol. 34, no. 8, 1986, pages 3440-3444, XP001154070 ISSN: 0009-2363 * the whole document * --- | 1-12 | |
| A | EP 0 930 311 A (DAISOW CO LTD) 21 July 1999 (1999-07-21) * the whole document * ----- | 1-12 | |
| | | | TECHNICAL FIELDS SEARCHED (Int.Cl.7) |

The present search report has been drawn up for all claims

| Place of search | Date of completion of the search | Examiner |
|---|---|---|
| MUNICH | 18 December 2003 | Pinheiro Vieira, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 03 02 4203

This annex lists the patent family members relating to the patent documents cited in the above-mentioned European search report.
The members are as contained in the European Patent Office EDP file on
The European Patent Office is in no way liable for these particulars which are merely given for the purpose of information.

18-12-2003

| Patent document cited in search report | Publication date | Patent family member(s) | Publication date |
|---|---|---|---|
| EP 0556909 A | 25-08-1993 | IT 1254463 B | 25-09-1995 |
| | | AT 161290 T | 15-01-1998 |
| | | CA 2089626 A1 | 18-08-1993 |
| | | DE 69315720 D1 | 29-01-1998 |
| | | DE 69315720 T2 | 23-07-1998 |
| | | DK 556909 T3 | 24-08-1998 |
| | | EP 0556909 A1 | 25-08-1993 |
| | | ES 2111124 T3 | 01-03-1998 |
| | | JP 3195108 B2 | 06-08-2001 |
| | | JP 6007195 A | 18-01-1994 |
| | | US 5332674 A | 26-07-1994 |
| EP 0930311 A | 21-07-1999 | AU 4136597 A | 02-04-1998 |
| | | EP 0930311 A1 | 21-07-1999 |
| | | US 6143908 A | 07-11-2000 |
| | | WO 9811087 A1 | 19-03-1998 |
| | | TW 420673 B | 01-02-2001 |

EPO FORM P0459

For more details about this annex : see Official Journal of the European Patent Office, No. 12/82